**Europäisches Patentamt**

**European Patent Office**

**Office européen des brevets**

(11) Veröffentlichungsnummer: **0 264 567 B1**

# EUROPÄISCHE PATENTSCHRIFT

(45) Veröffentlichungstag der Patentschrift: **21.11.91**

(51) Int. Cl.⁵: **C12M 1/00, C02F 3/10**

(21) Anmeldenummer: **87111896.4**

(22) Anmeldetag: **17.08.87**

(54) **Bio-Reaktor.**

(30) Priorität: **28.08.86 DE 3629299**

(43) Veröffentlichungstag der Anmeldung:
**27.04.88 Patentblatt 88/17**

(45) Bekanntmachung des Hinweises auf die
Patenterteilung:
**21.11.91 Patentblatt 91/47**

(84) Benannte Vertragsstaaten:
**AT CH DE FR IT LI SE**

(56) Entgegenhaltungen:
**AT-A- 361 015**
**DE-A- 2 155 631**
**DE-B- 1 246 610**
**FR-A- 1 422 447**

(73) Patentinhaber: **SIEMENS AKTIENGESELL-
SCHAFT**
**Wittelsbacherplatz 2**
**W-8000 München 2(DE)**

(72) Erfinder: **Flaig, Hans-Dieter, Dipl.-Ing.**
**Am Steinberg 22**
**W-6057 Dietzenbach(DE)**

Rank Xerox (UK) Business Services

## Beschreibung

Die Erfindung betrifft einen Bio-Reaktor mit einem Reaktortank und mit mehreren darin getrennt voneinander angeordneten Reaktormodulen, die von einer in den Reaktortank führenden Rohwasser-Leitung gemeinsam gespeist werden.

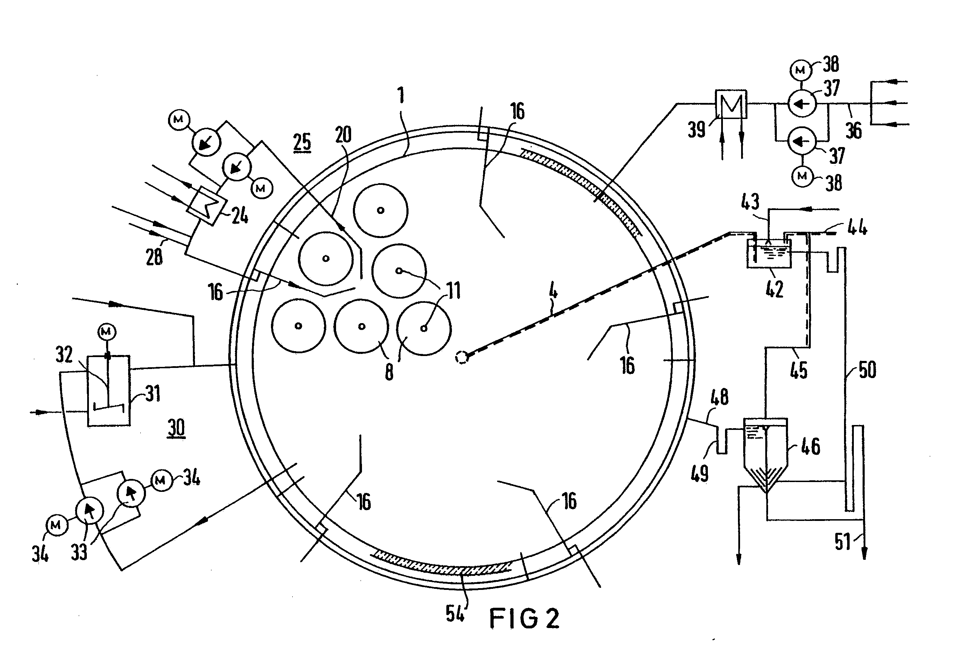

Bei solchen, z.B. aus der FR-PS 16 03 547 bekannten Bio-Reaktoren hat man sich bisher bemüht, den Raum im Reaktortank für die biologischen Vorgänge vollständig auszunutzen. Dies geschieht bei dem Bio-Reaktor nach der genannten Patentschrift dadurch, daß mit einer Luftleitung unten in den Reaktortank eingeleitete Luft in einer ersten Zone nach oben steigt, die von vielen parallel angeordneten zylindrischen Hüllen gebildet wird. Außerhalb der Hüllen soll das in etwa mittlerer Höhe des Reaktortanks eingeleitete Substrat wieder nach unten strömen, damit die genannten zylindrischen Hüllen mehrfach durchlaufen werden. In der FR-PS 16 03 547 ist nichts über die Querschnittsverhältnisse zwischen der Summe der einzelnen Hüllen und dem Reaktortank gesagt. Aus der Figur 2 läßt sich jedoch abschätzen, daß der Querschnitt der Hüllen mindestens die Hälfte des Querschnittes des Reaktorstanks beträgt.

Die Erfindung geht demgegenüber von der Aufgabe aus, einen Bio-Reaktor zu schaffen, der eine vereinfachte und verbesserte Verfahrensweise ermöglicht. Damit ist gemeint, daß der neue Bio-Reaktor mit geringem apparativem Aufwand in einem optimalen Temperaturbereich arbeitet.

Erfindungsgemäß ist ein Bio-Reaktor der eingangs genannten Art so ausgebildet, daß die Reaktormodulen zusammen höchstens etwa die Hälfte des Volumens des Reaktortanks aufweisen und daß an dem der Rohwasser-Leitung gegenüberliegenden Ende des Reaktortanks ein Leitungsanschluß vorgesehen ist, der als Schleife über eine Pumpe und einen Wärmetauscher zum rohwasserseitigen Ende des Reaktortanks führt.

Bei der Erfindung dient der Reaktortank zugleich als Vorratsbehälter für das sogenannte Substrat oder Rohwasser. Dies erspart einen besonderen Behälter für die Rohwasserspeicherung und ergibt vor allen Dingen zugleich die Möglichkeit, mit dem großen Flüssigkeitsvolumen eine einfache Temperaturregelung zu erreichen. Der Reaktortank mit seinem großen Flüssigkeitsvolumen wirkt nämlich als Wärmespeicher, der eine viel kleinere wärmeabgebende Oberfläche als die einzelne Module hat. Deshalb kann mit einem Wärmetauscher und einer relativ kleinen Pumpe leicht die für den biologischen Vorgang gewünschte Temperatur erreicht und eingehalten werden.

Der Wärmetauscher kann mit einer sperrbaren Leitung überbrückt sein, um mit der den Wärmetauscher zugeordneten Pumpe auch eine temperaturunabhängige Strömung durch den Reaktortank zu erzeugen.

Im Interesse einer großen Ausfallsicherheit ist es günstig, wenn einem Wärmetauscher zwei parallel arbeitende Pumpen zugeordnet sind. Ebenso kann der Reaktortank auch mehrere am Umfang verteilte Schleifen aufweisen, um eine gleichmäßige Durchströmung und damit eine gleichmäßige Temperierung zu erreichen.

Der Reaktortank kann ferner vorteilhaft an seiner Oberseite Stelleinrichtungen aufweisen, mit denen Stellglieder zur Steuerung des Durchsatzes durch die Module verbunden sind. Mit solche Stelleinrichtungen, die durch die vorzugsweise begehbare Decke des Reaktortanks hindurchragen, können z.B. Böden oder Siebe verstellt oder Drosseleinrichtungen geöffnet bzw. geschlossen werden.

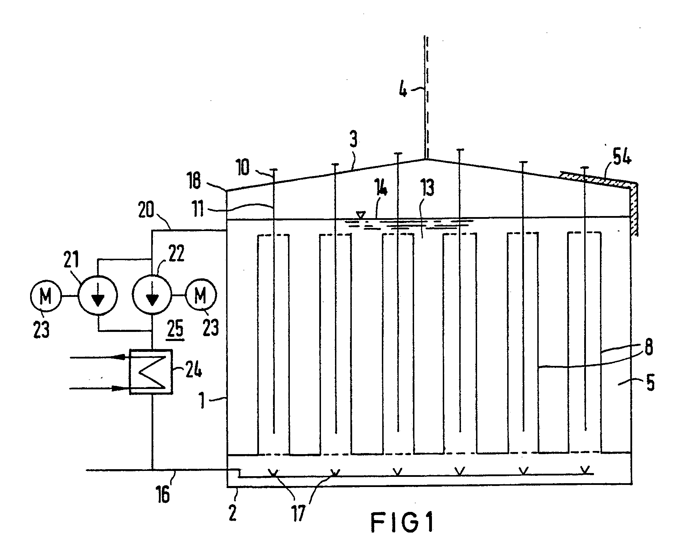

Zur näheren Erläuterung der Erfindung wird anhand der Figuren 1 und 2 ein Ausführungsbeispiel beschrieben, das in einer Seitenansicht und einer Draufsicht auf den Reaktortank dargestellt ist. In den beiden Figuren sind ferner in einem Rohrleitungsplan die mit dem Reaktortank verbundenen Einrichtungen zur Einspeisung und Austragung der Prozeßmedien zu sehen.

Der erfindungsgemäße Reaktortank 1 ist ein Stahl- oder Betonbehälter mit z.B. 2000 m³ Inhalt. Er hat eine zylindrische Form von 16 m Durchmesser mit einem ebenen Boden 2 und einem konischen Deckel 3, an dessen Spitze eine Gasauslaßleitung 4 vorgesehen ist. Die Höhe des Zylinders beträgt 10 m.

Im Inneren 5 des Reaktortanks 1 sind 30 Module 8 vorgesehen. Dies sind zylindrische Rohre mit einem Durchmesser von 2 m, d.h. höchstens einem Achtel des Durchmesser des Reaktortanks 1. Die Rohre 8 enthalten in bekannter Weise Böden, insbesondere Siebböden, auf denen geeigneten Betten zur Einbringungen von Biokulturen vorgesehen sind. Die Betten sind mit Stelleinrichtungen verstellbar, die aus dem Deckel 3 nach oben herausragen und über Stellglieder 11 mit den einzelnen Böden verbunden sind. Es müssen nicht alle Böden verstellbar sein. Die Verstellung kann zugleich den Durchsatz durch die Module 8 verändern.

Die Figur 2 läßt erkennen, daß die Module 8, die nur für einen Sektor mit etwa einem Fünftel des Querschnittes des Reaktortanks 1 dargestellt sind, weniger als die Hälfte des Tankquerschnittes bedecken. Das Restvolumen des Reaktortanks 1, also mehr als 1000 m³, dient als Speicher für das Rohwasser 13, dessen Flüssigkeitsstand bei 14 angedeutet ist. Das Rohwasser 13 wird über eine Leitung 16 in den Reaktortank 1 geführt, die im Bereich des Bodens 2 verläuft. Die Leitung 16 ist mit Auslaßöffnungen 17 versehen, die den einzelnen Modulen 8 zugeordnet sind.

An dem der Leitung 16 gegenüberliegenden, nämlich oberen Ende 18 des Reaktortanks 1 ist ein Leitungsanschluß 20 angeordnet, der über zwei parallele Pumpen 21 und 22 mit Elektromotoren 23 zu einem Wärmetauscher 24 führt. Pumpe und Wärmetauscher bilden sich somit eine Schleife 25, die von der Oberseite des Reaktortanks 1 zum rohwasserseitigen Einlaß 6 führt.

Die Figur 2 zeigt, daß um den Umfang des Reaktortanks 1 fünf Schleifen 25 verteilt angeordnet sind, die jeweils gleich ausgebildet sind. Jeder der fünf Schleifen umfaßt sechs Module 8, die über den Querschnitt des Reaktortanks in einem Sektor von 72° gleichmäßig verteilt sind. In der Figur 2 ist für vier der fünf Sektoren nur jeweils die Einlaßleitung 16 angedeutet.

In Figur 2 ist ferner angedeutet, daß an jeder Schleife ein Einlaß 28 für Natronlauge vorgesehen ist, die zur Steuerung und pH-Werteinstellung des anaeroben Reinigungsprozesses eingespeist werden kann. Die Natronlauge kann mit einer weiteren Schleife 30 konditioniert werden, die einen Druck-Behälter 31 mit einem Rührwerk 32 und zwei parallel liegende Pumpen 33 mit Elektromotorern 34 umfaßt.

Zur zusätzlichen Einspeisung von Substrat, also unabhängig von der Umwälzung in den Schleifen 25 ist eine Substratleitung 36 vorgesehen, die mit zwei Pumpen 37 mit Elektromotoren 38 versehen ist und über einen Wärmetauscher 39 in den Reaktortank 1 führt.

Die in der Mitte des Deckels 3 angeschlossene Abgasleitung 4 für das Bio-Gas führt über einen Schaumabscheider 42, der mit einer Leitung 43 zur Einspeisung eines Antischaummittels versehen sein kann. Aus der Auslaßleitung 44 abgehende Flüssigkeit kann über eine Leitung in einen Schlammabscheider 46 abgeleitet werden, der über eine Leitung 48 mit einem U-Rohr 49 an den Reaktortank 1 angeschlossen ist. In den Schlammabscheider 46 führt ferner eine Leitung 50, die aus dem Wasserbehälter des Schaumabscheiders 42 kommt und an die gleiche Reinwasserleitung 51 angeschlossen ist, an die auch der Schlammabscheider 46 angeschlossen ist.

Das Ausführungsbeispiel läßt deutlich erkennen, daß der Reaktortank 1 zugleich als Rohwasser-Vorlagebehälter genutzt wird. Er hat bei einer Großanlage mit den genannten 2000 m³ Inhalt nur eine wesentlich kleinere Oberfläche als die 30 Module 8, die jeweils 2 m Durchmesser haben. Ferner läßt sich mit der neuen Bauweise die Temperatur mit einfachen Mitteln steuern, weil das Rohwasservolumen als Wärmespeicher dient. Eine geeignete Temperatur beträgt z.B. 37° C. Dabei kann der Reaktortank 1 noch mit einer Wärmeisolierung 54 versehen sein, wenn sonst insbesondere bei Winterbetrieb eine zu starke Abkühlung zu befürchten ist.

Trotz der gemeinsamen Unterbringung der Module 8 Reaktortank 1 ist ein Rückspülen und Anheben des Bettes jedes einzelnen Modules 8 möglich. Durch die Verstell-und Abschlußmöglichkeiten der einzelnen Bio-Reaktoren kann jedes Modul 8 einzeln zurückgespült werden. Dabei kann die Strömungsgeschwindigkeit durch die verschie-denen Böden soweit gedrosselt werden, daß beispielsweise mit 150-facher Umlaufmenge zurückgespült wird.

Die Rückspülzeit liegt zwischen 20-30 Min.. Während dieser Rückspülzeit können die übrigen Module 8 weiterhin in Betriebsstellung stehen. Dies ist ein Vorteil gegenüber wenigen großen Modulen, deren Rückspülung die Gesamtkapazität erheblich reduzieren würde.

Um die Bakterien der Bio-Kulturen günstig mit Nährstoffen zu versorgen, wird bei der Erfindung mit 10-15-facher Umwälzung gefahren und Nährlösung in den Umwälzkreislauf eingespeist.

Da die Rohwässer bisweilen mit Temperaturen von z.B. 45° C und höher anfallen, können sie durch Wärmetauscher auch auf ca. 37-39° gekühlt werden. Hier ist eine Kombination der Wärmeaustauscher denkbar, bei der im Wärmetauscher 24 das Erwärmen und im Wärmetauscher 39 das Kühlen vorgenommen wird.

**Patentansprüche**

1. Bio-Reaktor mit einem Reaktortank und mit mehreren darin getrennt voneinander angeordneten Reaktormodulen, die von einer in den Reaktortank führenden Rohwasser-Leitung gemeinsam gespeist werden, **dadurch gekennzeichnet,** daß die Reaktormodule (8) zusammen höchstens etwa die Hälfte des Volumens des Reaktortanks (1) aufweisen und daß an dem der RohwasserLeitung (16) gegenüberliegenden Ende (18) des Reaktortanks (1) ein Leitungsanschluß (20) vorgesehen ist, der als Schleife (25) über eine Pumpe (21) und einen Wärmetauscher (24) zum rohwasserseitigen Ende des Reaktortanks (1) führt.

2. Bio-Reaktor nach Anspruch 1, **dadurch gekennzeichnet,** daß der Wärmetauscher (24) mit einer sperrbaren Leitung überbrückt ist.

3. Bio-Reaktor nach Anspruch 1 oder 2, **dadurch gekennzeichnet,** daß einem Wärmetauscher (24) zwei parallel arbeitende Pumpen (21,22) zugeordnet sind.

4. Bio-Reaktor nach Anspruch 1,2 oder 3, **dadurch gekennzeichnet,** daß am Umfang des Reaktortanks (1) mehrere Schleifen (25) verteilt angeordnet sind.

5. Bio-Reaktor nach einem der Ansprüche 1 bis 4, **dadurch gekennzeichnet,** daß der Reaktortank (1) an seiner Oberseite (3) Stelleinrichtungen (10) aufweist, mit denen Stellglieder (11) zur Steuerung des Durchsatzes durch die Module (8) verbunden sind.

## Claims

1. A bio-reactor with a reactor tank and with several reactor units arranged therein and separated from each other, which are fed together from an untreated water line leading into the reactor tank, characterised in that the reactor units (8) in total take up at the most approximately half of the volume of the reactor tank (1), and in that on that end (18) of the reactor tank (1), opposite the untreated water line (16), there is provided a line connection (20), which forms a loop (25) leading by way of a pump (21) and a heat exchanger (24) to the untreated water end of the reactor tank (1).

2. A bio-reactor according to claim 1, characterised in that the heat exchanger (24) is bridged with a blockable line.

3. A bio-reactor according to claim 1 or 2, characterised in that associated with the heat exchanger (24) there are two pumps (21,22) operating in parallel.

4. A bio-reactor according to claim 1,2 or 3, characterised in that distributed over the periphery of the reactor tank (1), there are arranged several loops (25).

5. A bio-reactor according to one of claims 1 to 4, characterised in that the reactor tank (1) on its upper side (3) has adjusting devices (10), to which are connected adjusting members (11) for controlling the throughput through the units (8).

## Revendications

1. Bioréacteur comprenant une cuve de réacteur et plusieurs modules de réacteur, qui sont disposés en étant séparés les uns des autres et qui sont alimentés en commun par un conduit pour de l'eau brute menant à la cuve du réacteur, caractérisé en ce que les modules de réacteur (8) représentent ensemble au plus environ la moitié du volume de la cuve du réacteur (1) et en ce qu'à l'extrémité (18) de la cuve du réacteur (1), qui est opposée au conduit (16) pour l'eau brute, est prévu un raccord de conduit (20) qui mène, par un circuit fermé (25) passant par une pompe (21) et par un échangeur de chaleur (24), à l'extrémité de la cuve du réacteur (1) qui se trouve du côté de l'eau brute.

2. Bioréacteur suivant la revendication 1, caractérisé en ce que l'échangeur de chaleur (24) est court-circuité par un conduit qui peut être fermé.

3. Bioréacteur suivant la revendication 1 ou 2, caractérisé en ce que deux pompes (21,22) fonctionnant en parallèle sont associées à un échangeur de chaleur (24).

4. Bioréacteur suivant la revendication 1, 2 ou 3, caractérisé en ce que plusieurs circuits fermés (25) sont répartis sur le pourtour de la cuve du réacteur (1).

5. Bioréacteur suivant l'une des revendications 1 à 4, caractérisé en ce que la cuve du réacteur (1) comporte, sur son côté supérieur (3), des dispositifs de réglage (10) auxquels sont reliés des organes de réglage (11) destinés à commander le débit dans les modules (8).

FIG 1

FIG 2